# FASCICULE DE BREVET EUROPEEN

(11) **EP 0 721 472 B1**
(45) Date de publication et mention de la délivrance du brevet: **11.06.1997**
(21) Numéro de dépôt: 94928936.7
(22) Date de dépôt: 30.09.1994
(51) Int. Cl.: C08F 2/08, C09D 133/06

(54) **MICRODISPERSIONS STABLES ET MICROGELS A BASE DE POLYMERES ACRYLIQUES, LEUR PROCEDE D'OBTENTION ET COMPOSITIONS, NOTAMMENT COSMETIQUES, LES CONTENANT**
STABILE MIKRODISPERSIONEN UND MIKROGELE AUF BASIS VON ACRYLATPOLYMERE, VERFAHREN ZU DEREN HERSTELLUNG UND KOSMETISCHE ZUSAMMENSETZUNGEN, DIESE ENTHALTEND
STABLE MICRODISPERSION AND MICROGELS BASED ON ACRYLIC POLYMERS, METHOD FOR OBTAINING THEM AND COMPOSITIONS, PARTICULARLY COSMETIC COMPOSITIONS CONTAINING THEM

(30) Priorité: 01.10.1993 FR 9311705
(43) Date de publication de la demande: 17.07.1996
(73) Titulaire: LVMH RECHERCHE, 92752 Nanterre (FR)
(72) Inventeur: KUENTZ, Annie, F-68100 Mulhouse (FR); RIESS, Henri-Gérard, F-68200 Mulhouse (FR); MEYBECK, Alain, F-92400 Courbevoie (FR); TRANCHANT, Jean-François, F-45760 Boigny-sur-Bionne (FR)
(74) Mandataire: Giraud, Françoise
(86) Numéro de dépôt international: FR9401145
(87) Numéro de publication internationale: WO9509874

(56) Documents cités:
- DE-B- 1 201 064
- EUROPEAN POLYMER JOURNAL, vol.23, no.2, 1987, OXFORD pages 173 - 175 J.V.DAWKINS 'NON-AQUEOUS POLY(METHYL METHACRYLATE DISPERSIONS: RADICAL DISPERSION POLYMERIZATION IN THE PRESENCE OF THE DIBLOCK COPOLYMER POLY(STYRENE-B-METHYL METHACRYLATE)' cité dans la demande

## Description

La présente invention concerne de nouvelles microdispersions stables de particules polymériques, les microgels obtenus à partir de ces dispersions ainsi que des compositions renfermant ces microdispersions ou microgels, notamment des compositions cosmétiques. Elle concerne également un procédé pour préparer ces dispersions et microgels.

Par microdispersion au sens de l'invention, on entend une dispersion de particules de taille pouvant aller jusqu'à 120nm et de préférence inférieure à 100 nm. Le polymère constituant ces particules est sous forme précipitée dans le milieu réactionnel, les particules étant stabilisées par un copolymère.

En accord avec Antonietti et coll. dans "Makromol. Chem., Makromol. Symp." 30, 81-93 (1989), on définit selon l'invention un microgel comme une microparticule de polymère réticulé, sous forme gonflée, en dispersion dans un solvant organique, dont la taille, gouvernée par le taux de gonflement, est fonction de la densité de réticulation. Le polymère qui le compose, sous la forme non réticulée, est soluble dans ce même solvant, seule la réticulation empêche les particules d'être dissoutes.

La différence essentielle entre une microdispersion et un microgel est le fait que le premier se trouve sous forme précipitée dans le milieu réactionnel formé par un solvant ne solubilisant pas le polymère, tandis que le microgel est sous forme gonflée dans des solvants du polymère.

A titre d'exemple, une microdispersion de PMMA préparée en milieu réactionnel alcoolique se trouve sous forme précipitée, du fait que l'alcool n'est pas un solvant du PMMA, la stabilisation de la microdispersion sous forme précipitée étant assurée par le copolymère-bloc. Ces mêmes particules étant réticulées, elles permettent d'obtenir des microgels sous forme gonflée lorsqu'on les transfère dans un solvant du PMMA tel que l'acétate de butyle.

On a décrit dans un certain nombre de publications des procédés de préparation de microgels acryliques dans des hydrocarbures aliphatiques soit seuls, soit en mélange avec d'autres hydrocarbures ou avec des alcools.

Ainsi, le brevet US 4 558 075 décrit la préparation de microparticules acryliques en présence de copolymères statistiques PGMA-PMMA-PMAA dans un milieu réactionnel constitué d'heptane et leur application dans le domaine de la peinture automobile avec des paillettes d'aluminium. Les particules polymériques ont des dimensions comprises entre 0,01 et 10 µ.

DH EVERETT, JF STAGEMAN (Discus. Faraday Soc. 65, 230, 1978) ont décrit la préparation de dispersions de polystyrène ou de PMMA en présence de copolymères triblocs PDMS-b-PS-b-PDMS en utilisant des alcanes comme milieu réactionnel. Les particules polymériques obtenues selon ce document ont des dimensions de l'ordre de 0,1 µ.

JV DAWKINS, SA SHAKIR, TG CROUCHER, Europ. Polym. J. 23, 173-175, 1987 ont décrit la synthèse de dispersions de PMMA dans le cyclohexane en présence de copolymères PS-PMMA comme agents stabilisants.

Le brevet allemand DE 3 439 128 décrit la préparation de microgels acryliques stabilisés soit par des polyesters soit par des copolymères de type "polyacide hydroxystéarique-PMMA-PAA" dans un milieu réactionnel constitué d'eau ou d'hydrocarbures aliphatiques et leurs applications pour préparer des laques métalliques à base de microgels aqueux ou des laques blanches à base d'oxyde de titane dans le cas des microgels dans les hydrocarbures aliphatiques. Les dimensions des particules polymériques décrites dans ce document sont de l'ordre de 0,01 à 10 µ.

D'autres publications citent l'utilisation de mélanges d'hydrocarbures et d'alcools comme milieu réactionnel. C'est le cas du brevet européen EP 251 553 dans lequel on prépare des microgels acryliques en présence d'agents stabilisants copolymérisables que l'on copolymérise avec des monomères acryliques pour faire un core-shell. Les dispersions obtenues sont utilisables pour la peinture des métaux.

C'est également le cas de la publication de CH. BAMFORD et al, J. Appl. Polym. Sci., 25, 2559-2566, 1980 qui décrit, elle aussi, des microgels dans un milieu solvant constitué d'un mélange d'alcool et d'hydrocarbure sans toutefois recourir à l'utilisation d'un agent stabilisant tensioactif.

Ainsi donc, il apparaît que, en ce qui concerne les microgels acryliques, les copolymères stabilisants sont en général des copolymères greffés ou statistiques à base de polyméthacrylate de méthyle. La publication de JV DAWKINS et al , Euro. Polym. J. 23, 173-175, 1987 utilise, quant à elle, des copolymères-blocs PMMA-b-PS comme agents stabilisants de dispersions de polyméthacrylate de méthyle.

Le brevet anglais GB 941 305 décrit la préparation de polymères acryliques stabilisés par des copolymères-blocs également acryliques et, ce, dans l'alcool éthylique. La partie stabilisatrice du copolymère employé dans ce brevet est composée de polyacide méthacrylique. Les tailles des particules constituant les dispersions décrites dans le brevet GB 941 305 sont de l'ordre de 0,2 µ et plus. Par ailleurs les dispersions obtenues en procédant selon l'enseignement de ce brevet ont des extraits secs compris entre 25 et 65 %.

Au cours de ses recherches, la demanderesse a découvert qu'en utilisant comme agent stabilisant un copolymère-bloc à base de polyméthacrylate de méthyle (PMMA) et de polyacrylate de tertiobutyle (PAtBu), on pouvait préparer en milieu alcoolique des microdispersions de particules de polymères acryliques de dimensions beaucoup plus faibles que celles décrites jusqu'à présent, inférieures à 120 nm et généralement voisines de 80 nm, ce qui présente un grand intérêt pour la stabilité de ladite dispersion.

Ces particules présentent en plus un faible indice de polydispersité en taille, caractérisé par le rapport Dw/Dn, où Dw est la taille moyenne en masse et Dn la taille moyenne en nombre.

Compte tenu de leur faible taille, ces particules ont une surface spécifique importante, ce qui peut être un élément favorable dans des compositions comportant des pigments ou des charges.

Par ailleurs, les microdispersions de l'invention présentent également l'avantage de pouvoir être concentrées, voire même séchées et redispersées dans un solvant, par exemple un alcool tout en conservant les mêmes caractéristiques.

Un autre avantage des microdispersions selon l'invention est qu'elles sont compatibles avec les esters pour former des microgels.

Les avantages et caractéristiques de l'invention apparaissent dans la description qui va suivre.

Selon une de ces caractéristiques essentielles, l'invention concerne un procédé de préparation d'une microdispersion stable de particules constituées de polymères acryliques dans un solvant organique utilisé comme milieu réactionnel en présence d'un agent stabilisant constitué d'un copolymère-bloc, caractérisé en ce que ledit polymère acrylique est obtenu par polymérisation radicalaire d'au moins un monomère acrylique en présence d'un copolymère-bloc à base de polyméthacrylate de méthyle (PMMA) et de polyacrylate de tertiobutyle (PAtBu).

Ainsi le procédé de l'invention consiste à préparer un polymère acrylique par un procédé connu de polymérisation radicalaire dans un solvant et à réaliser cette polymérisation en présence d'un copolymère-bloc réalisé entre le PMMA et le PAtBu.

Ce copolymère-bloc a avantageusement une masse moléculaire comprise entre 20 000 et 500 000, de préférence entre 40 000 et 200 000.

Il peut s'agir soit d'un copolymère dibloc symboliquement représenté par PMMA-b-PAtBu, soit d'un copolymère de type tribloc symbolisé par PAtBu-b-PMMA-b-PAtBu.

Dans le cas du dibloc, le pourcentage massique du bloc polyacrylate de tertiobutyle sera compris entre 10 et 90 %, mais de préférence proche de 50 %.

La proportion de copolymère introduite, donnée par rapport à la masse totale de monomères introduite sera supérieure ou égale à 5 %, mais de préférence de l'ordre de 10 %.

Selon une variante avantageuse du procédé, le monomère acrylique est choisi dans la famille des alkylacrylates et alkylméthacrylates présentant des groupements alkyles en C₁ à C₁₈, linéaires ou ramifiés, ou leurs mélanges. A titre d'exemples de groupement alkyle, on citera les groupements méthyle, butyle, lauryle. A titre d'exemples préférés de monomères acryliques, on citera le méthacrylate de méthyle, l'acrylate de butyle ou un mélange de ces deux monomères. On citera également les mélanges de méthacrylate de méthyle et de méthacrylate de lauryle.

Ces monomères acryliques seront avantageusement copolymérisés selon le procédé de l'invention avec un monomère difonctionnel.

A titre d'exemple de monomère difonctionnel, on citera le divinylbenzène, les monomères diacryliques ou diméthacryliques, par exemple le diméthacrylate de butanediol, le diméthacrylate d'éthylèneglycol, le diméthacrylate de diéthylèneglycol, le diméthacrylate de tétraéthylèneglycol.

Un monomère difonctionnel préféré selon l'invention est le diméthacrylate de butanediol (BDMA).

La quantité de copolymère-bloc utilisé pour former les microdispersions de la présente invention est avantageusement comprise entre 1 et 50 % en poids, de préférence entre 5 et 30 %, par rapport à l'ensemble des monomères mis en oeuvre pour préparer ledit polymère acrylique.

Le milieu réactionnel utilisé est un alcool, en particulier un alcool en C₁ à C₄, de préférence l'éthanol ou un mélange d'alcools précédemment définis, par exemple un mélange d'éthanol et d'isopropanol.

On peut également utiliser comme milieu réactionnel tout solvant qui solubilise la chaîne PAtBu du copolymère stabilisateur, et qui précipite la chaîne PMMA de ce même copolymère ainsi que le polymère acrylique formé. On peut utiliser en particulier un milieu riche en alcool, par exemple un mélange d'éthanol avec 20 % en volume d'acétate de butyle, ou un mélange d'éthanol avec 20 % en volume d'acétate d'éthyle.

La polymérisation radicalaire est réalisée de façon classique en présence d'un amorceur de polymérisation radicalaire constitué par un amorceur organo-soluble choisi de préférence dans la famille des amorceurs azoïques et des peroxydes.

A titre d'exemples d'amorceurs préférés on citera l'azobisisobutyronitrile et le peroxyde de benzoyle.

De façon avantageuse, la polymérisation radicalaire est réalisée en plusieurs étapes comprenant la préparation d'une semence suivie d'un ajout progressif du reste des produits.

La semence sera de préférence constituée d'environ 20 % du mélange monomères/réticulant, et d'environ 50 % de l'amorceur. Ce mélange sera ajouté à la solution micellaire préformée du copolymère dans l'alcool, à la température de polymérisation, que l'on réalise de préférence à environ 5 à 10°C en dessous du point d'ébullition du solvant constituant le milieu réactionnel, et on laisse se dérouler la polymérisation pendant environ 4 h.

A la semence ainsi formée, on ajoutera lentement le reste des constituants, mais de préférence tout l'amorceur restant dissous dans de l'alcool, et les monomères pendant une durée comprise avantageusement entre 2 et 4 h.

La synthèse sera arrêtée après une période de polymérisation supplémentaire de l'ordre de 16 h.

Les microdispersions de polymères acryliques décrites précédemment présentent des dimensions de particules et une monodispersité jamais atteintes jusqu'à présent pour des dispersions en milieu alcoolique. Elles constituent des produits industriels nouveaux en soi.

Ainsi selon un autre de ses aspects l'invention concerne les dispersions de polymères acryliques susceptibles d'être obtenues par le procédé décrit précédemment.

Les dispersions sont composées de particules de dimensions inférieures à 120 nm, généralement voisines de 80 nm, et d'un faible indice de polydispersité (entre 1,1 et 1,2)

Par ailleurs, ces microdispersions présentent les avantages suivants :
- les microdispersions sont compatibles avec les esters, tels que l'acétate de butyle, en formant des microgels ;
- les produits obtenus sont compatibles avec la nitrocellulose dissoute dans de l'acétate de butyle en donnant un film transparent et brillant ;
- le polymère obtenu ne comporte pas de fonctions acides, celles-ci étant en général préjudiciables pour les formulations à base de nitrocellulose ;
- la réticulation des particules permet un ajustement des caractéristiques rhéologiques ;
- les microdispersions ainsi préparées permettent d'obtenir des vernis à ongles en milieu alcool.

Ainsi, selon un autre de ses aspects, l'invention concerne les microgels obtenus à partir des microdispersions de l'invention. Ces microgels sont obtenus plus précisément en transférant les particules dans un solvant du PMMA soit après séchage préalable des microdispersions, soit par ajout de ce même solvant à la microdispersion préalablement concentrée.

A titre de solvant, on citera les solvants aromatiques, les solvants chlorés tels que le chloroforme ou le chlorure de méthylène, des cétones et des esters tels que les acétates d'alkyle en C₂ à C₄, plus particulièrement l'acétate de butyle et l'acétate d'éthyle.

Principalement, pour éviter d'obtenir des microgels de trop forte viscosité, et donc pour faciliter leur utilisation, la concentration pondérale de polymères dans les microgels, en particulier lorsque ceux-ci sont destinés à la formulation de vernis, tels que les vernis à ongles, ne dépasse pas en général 30 % environ. De préférence, cette proportion est comprise entre 10 % et 20 % environ.

Les dispersions et microgels de l'invention s'avèrent particulièrement utiles dans les domaines de la peinture et de la cosmétique, où ils permettent d'ajuster les caractéristiques rhéologiques. Il est en effet possible de mélanger la microdispersion à des formulations à base de nitrocellulose à extrait sec élevé, sans augmenter notablement la viscosité du système qui en résulte.

Le fait que les produits soient compatibles avec la nitrocellulose dissoute dans l'acétate de butyle en donnant un film transparent et brillant permet d'envisager leur utilisation pour faire des vernis, particulièrement des vernis à ongles.

Plus précisément, l'utilisation des microgels de l'invention dans des formulations de vernis et en particulier de vernis à ongles présente en particulier les avantages suivants :
- elle permet une amélioration des propriétés rhéologiques permettant, en particulier, d'éviter la précipitation des pigments, et une amélioration de la reproductibilité de ces propriétés. Ceci permet, en particulier, de diminuer sensiblement, voire même de supprimer, la quantité de bentone généralement utilisée à cet effet mais dont les inconvénients sont bien connus,
- elle permet d'augmenter l'extrait sec du film constitué par le vernis, sans pour autant augmenter sensiblement la viscosité du vernis,
- elle confère plus de brillant au film,
- elle renforce l'effet thixotropique apporté par la bentone en milieu acétate.

La proportion pondérale de microgel selon l'invention, dans la composition finale du vernis, peut s'élever jusqu'à 30 % environ, par exemple dans le cas où on chercherait à diminuer, voire supprimer la quantité de nitrocellulose. Mais, en général, on préfère utiliser des proportions comprises entre 5 et 20 % en poids environ.

Les exemples suivants sont donnés à titre illustratif de l'invention.

### Exemples

### Exemple 1 : préparation d'une microdispersion selon l'invention

Le copolymère dibloc utilisé dans cet exemple (copolymère I) a une masse molaire moyenne en nombre de 90 000 et comprend 50 % en poids de PAtBu, les blocs PAtBu et PMMA ont des Mn de 45 000 chacun.

On porte 150g d'éthanol et 0,76g de copolymère à 65°C, sous agitation, dans un réacteur à double enveloppe équipé d'un réfrigérant et d'une arrivée d'azote pendant 30 minutes.

On ajoute ensuite 0,2g d'azobisisobutyronitrile (AIBN), 1,5g de méthacrylate de méthyle (MAM) et 0,075g de butanediol diméthacrylate (BDMA), que l'on laisse polymériser pendant 4 h.

On introduit 0,2 g d'AIBN dissous dans 20 ml d'éthanol, suivi d'un ajout de 5,70 g de MAM et 0,29 g de BDMA à une vitesse de 2,5 ml/h. A la fin de l'ajout, la polymérisation est poursuivie pendant environ 16 h. La microdispersion obtenue présente un extrait sec de 4,1 %, les particules ont une taille de 98 nm, et la valeur de Dw/Dn est de 1,12.

### Exemple 2 : Préparation d'une microdispersion

Le procédé de synthèse est le même que celui donné pour l'exemple 1, seules les proportions des divers constituants changent.
- préparation de la semence :

| | |
|---|---|
| 150 g | EtOH |
| 1,87 g | copolymère I |
| | |
| 0,2 g | AIBN |
| 0,53 g | ABu |
| 0,97 g | MAM |
| 0,075 g | BDMA |

puis ajout de 0,2 g AIBN dans 20 ml EtOH, suivi d'un ajout à 2,5 ml/h de :

| | |
|---|---|
| 2,0 g | ABu |
| 3,7 g | MAM |
| 0,29 g | BDMA |

La dispersion obtenue a un extrait sec de 4,7%, les tailles des particules obtenues sont de 86,5 nm, et la valeur de Dw/Dn est de 1,14.

### Exemple 3 : démontrant la possibilité d'utiliser un copolymère de masse moléculaire plus élevée

Le copolymère dibloc employé dans cet exemple (copolymère II) a une masse molaire moyenne en nombre de 150000, comprend 75 % en poids de PAtBu, les blocs PAtBu et PMMA ont des Mn de 112 500 et 37 500 respectivement.

Le mode opératoire n'est pas modifié, les proportions des divers constituants sont les suivantes :
- préparation de la semence :

| | |
|---|---|
| 150 g | EtOH |
| 1,87 g | copolymère II |
| 0,2 g | AIBN |
| 0,75 g | MAM |
| 0,75 g | ABu |
| 0,075 g | BDMA |

puis ajout de 0,2 g AIBN dans 20 ml EtOH, suivi d'un ajout à 2,5 ml/h de :

| | |
|---|---|
| 2,85 g | MAM |
| 2,85 g | ABu |
| 0,29 g | BDMA |

La dispersion obtenue présente un extrait sec final de 4,7 %, des tailles de particules de 94,3 nm et une valeur de Dw/Dn de 1,15.

### Exemple 4 : démontrant la possibilité d'utiliser un copolymère riche en PMMA

Le copolymère dibloc utilisé ici (copolymère III) a une masse molaire moyenne en nombre de 80 000, et comprend 35,4 % en poids de PAtBu, les blocs PAtBu et PMMA ont des Mn de 28 300 et 51 700 respectivement.

Le déroulement de la synthèse reste inchangé, les ingrédients introduits sont les suivants :
- préparation de la semence :

| | |
|---|---|
| 150 g | EtOH |
| 0,76 g | copolymère III |
| 0,2 g | AIBN |
| 0,75 g | ABu |
| 0,75 g | MAM |
| 0,075 g | BDMA |

puis ajout de 0,2 g AIBN dans 20 ml EtOH, suivi d'un ajout à 2,5 ml/h de :

| | |
|---|---|
| 2,85 g | MAM |
| 2,85 g | ABu |
| 0,29 g | BDMA |

La dispersion obtenue présente un taux de solide de 4,3 %, des tailles de particules de 81,3 nm, et un Dw/Dn de 1,14.

### Exemple 5 : démontrant l'influence de la masse molaire moyenne du copolymère-bloc

Le copolymère dibloc IV, utilisé pour cet exemple a un Mn total de 26 000, le pourcentage en masse du bloc PAtBu représentant 85 %.

Le procédé de synthèse est le même, les proportions engagées sont les suivantes :
- préparation de la semence :

| | |
|---|---|
| 250 g | EtOH |
| 3 g | copolymère IV |
| 1,40 g | MAM |
| 0,07 g | BDMA |
| 0,1 g | AIBN |

puis ajout de 0,4 g AIBN dans 20 ml EtOH, suivi d'un ajout à 2,5 ml/h de :

| | |
|---|---|
| 13,34 g | MAM |
| 0,29 g | BDMA |

La dispersion présente toutefois une stabilité sensiblement inférieure à celle des dispersions obtenues à l'aide des polymères de Mn ≥ à 40 000.

### Exemple 6 : préparation d'une microdispersion à partir d'un mélange de monomère acrylique

Le copolymère dibloc utilisé dans cet exemple a une masse molaire moyenne en nombre de 53 000 et comprend 85 % en poids de PAtBu et 15 % de PMMA.

On porte 150 g d'un mélange d'isopropanol/éthanol contenant 80 % d'isopropanol et 20 % d'éthanol et 0,76g de copolymère dibloc à 65°C, sous agitation, dans un réacteur à double enveloppe équipé d'un réfrigérant et d'une arrivée d'azote pendant 30 minutes.

On ajoute ensuite 0,2g d'AIBN, 1,62g de MAM et 0,18g de méthacrylate de lauryle (MAL) et 0,075g de BDMA. On laisse polymériser pendant 4 h.

On introduit 0,2 g d'AIBN dissous dans 20 ml de mélange isopropanol/éthanol à 80/20 et on ajoute 6,48 g de MAM, 0,72 g de MAL et 0,29 g de BDMA à une vitesse de 2,5 ml/h. A la fin de l'ajout, la polymérisation est poursuivie pendant environ 16 h. La microdispersion obtenue présente un extrait sec d'environ 5,5 %, les particules qui la constitue ont une taille d'environ 108 nm.

### Exemple 7 : démontrant la possibilité d'utiliser un copolymère tribloc du type PAtBu-b-PMMA-b-PAtBu

Le copolymère tribloc, copolymère V, a une masse molaire moyenne en nombre de 30 000, un pourcentage en PAtBu de 54 %, un Mn en PMMA de 14 000.

Le déroulement de la synthèse est le même que précédemment, les quantités introduites sont :
- préparation de la semence : 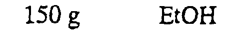 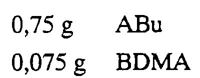
puis ajout de 0,2 g AIBN dans 20 ml EtOH, suivi d'un ajout à 2,5 ml/h de :

| | |
|---|---|
| 2,85 g | MAM |
| 2,85 g | ABu |
| 0,29 g | BDMA |

La dispersion obtenue présente un extrait sec de 4 %, des tailles de particules de 101 nm, et un Dw/Dn de 1,13.

### Exemple 8 : préparation des microgels et compatibilité avec la nitrocellulose

Plusieurs modes de préparation de microgels sont possibles. Le plus simple consiste à sécher complètement à l'évaporateur rotatif la microdispersion et à redisperser le polymère dans une quantité adéquate de solvant du PMMA, par exemple d'acétate de butyle pour obtenir le microgel à concentration voulue.

Un autre procédé d'obtention des microgels consiste à concentrer la microdispersion à l'évaporateur rotatif, et à rajouter directement une quantité de solvant du PMMA, par exemple d'acétate de butyle, nécessaire pour solubiliser le PMMA. On considère généralement que la quantité de solvant du PMMA doit représenter un volume de plus de 20 % du volume total de solvant.

Selon cette même méthode, on peut également ajouter directement à la microdispersion concentrée une solution de nitrocellulose dissoute dans de l'acétate de butyle.

A titre d'exemple, ce dernier procédé peut être illustré comme suit :

La microdispersion obtenue dans l'exemple 3 est concentrée à l'évaporateur rotatif à 40°C jusqu'à l'obtention d'une microdispersion de concentration de 20 % dans l'éthanol. Celle-ci présente une viscosité à 20°C de 7,0.10⁻³ Pa.s (7 cP) comparé à 1,2.10⁻³ Pa.s (1,2 cP) pour l'éthanol seul à la même température.

Le mélange en proportions égales de la même microdispersion à 20 % dans l'éthanol, avec une solution de nitrocellulose du type CA4 A20 à 20 % dans l'acétate de butyle de viscosité 17,9.10⁻³ Pa.s (17,9 cP), présente une viscosité de 31,6.10⁻³ Pa.s (31,6 cP).

Les solutions obtenues sont limpides et ne présentent pas de séparation de phase, même au stockage, ce qui démontre la compatibilité entre les microgels et la nitrocellulose.

### Exemple 9 : préparation de vernis à ongles

On prépare différents vernis à ongles par introduction dans une base nitrocellulosique d'un microgel selon l'invention obtenu à partir de la microdispersion de l'exemple 1 par dispersion du polymère dans l'acétate de butyle à l'aide d'un agitateur de type défloculeuse. Par rapport au poids total du vernis ainsi préparé, la proportion de microgel représente de 5 à 20 % environ.

Les bases nitrocellulosiques utilisées pour la préparation des vernis à ongles ont, par exemple, les compositions suivantes en pourcentages en poids :
- nitrocellulose 10 à 20
- résine arylsulfonamide (Santolite® ou Lustralite®) 10 à 15
- résine polyester 2 à 5
- solvants (mélange d'acétate de butyle, d'acétate d'éthyle et de toluène, comprenant moins de 40 % de toluène) 60 à 80
- plastifiant : 0,5 à 8
   . dibutylphtalate
   . Citroflex-A2®
   . camphre
- agent antisédimentant, par exemple bentone, 0 à 1,5
- pigments 0 à 2

### Exemple 10 : vernis à ongles

On prépare une formule de vernis à ongles selon l'exemple 9 comprenant les proportions suivantes en pourcentages en poids :
- nitrocellulose 15
- microgel selon l'invention 15
- dibutylphtalate 1
- solvant (composé de 55 % d'acétate d'éthyle,
   15 % d'acétate de butyle,
   30 % de toluène) 68
- pigments 1

## Revendications

1. Procédé de préparation d'une microdispersion stable de particules constituées de polymères acryliques dans un solvant organique utilisé comme milieu réactionnel en présence d'un agent stabilisants constitué d'un copolymère-bloc, caractérisé en ce que ledit polymère acrylique est obtenu par polymérisation radicalaire d'au moins un monomère acrylique en présence d'un copolymère-bloc à base de polyméthacrylate de méthyle (PMMA) et de polyacrylate de tertiobutyle (PAtBu).

2. Procédé selon la revendication 1, caractérisé en ce que la masse moléculaire dudit copolymère-bloc est comprise entre 20 000 et 500 000, de préférence entre 40 000 et 200 000.

3. Procédé selon l'une des revendications 1 ou 2, caractérisé en ce que le copolymère-bloc est un copolymère dibloc et en ce que le bloc PAtBu représente 10 à 90 % en poids, de préférence de l'ordre de 50 % dudit copolymère.

4. Procédé selon l'une des revendications 1 ou 2, caractérisé en ce que ledit copolymère-bloc est un copolymère de type tribloc PAtBu-b-PMMA-b-PAtBu.

5. Procédé selon l'une des revendications 1 à 4, caractérisé en ce que ledit monomère acrylique est un alkylacrylate ou un alkylméthacrylate, lesdits alkyles étant des alkyles en C₁ à C₁₈ linéaires ou ramifiés, ou un mélange desdits monomères.

6. Procédé selon l'une des revendications 1 à 5, caractérisé en ce que le polymère acrylique est un copolymère d'au moins un monomère acrylique et d'un monomère di-fonctionnel.

7. Procédé selon la revendication 6, caractérisé en ce que le monomère di-fonctionnel est le diméthacrylate de butanediol (BDMA).

8. Procédé selon l'une des revendications 1 à 7, caractérisé en ce que la quantité de copolymère-bloc est comprise entre 1 et 50 % en poids, de préférence entre 5 et 30 %, par rapport à l'ensemble des monomères mis en oeuvre pour préparer ledit polymère acrylique.

9. Procédé selon l'une des revendications 1 à 8, caractérisé en ce que le solvant est un alcool, en particulier en C₁ à C₄, un mélange d'alcools, en particulier un mélange d'éthanol et d'isopropanol, ou un milieu riche en alcool, par exemple un mélange d'éthanol et d'acétate de butyle ou tout solvant qui solubilise la chaîne PAtBu du copolymère stabilisateur et précipite la chaîne PMMA de ce même copolymère ainsi que le polymère acrylique.

10. Procédé selon la revendication 9, caractérisé en ce que ledit solvant est de l'éthanol.

11. Procédé selon l'une des revendications 1 à 10, caractérisé en ce que ladite polymérisation radicalaire est réalisée en présence d'un amorceur appartenant à la famille des azoïques et des peroxydes.

12. Procédé selon la revendication 11, caractérisé en ce que ledit amorceur est l'azobisisobutyronitrile ou le peroxyde de benzoyle.

13. Procédé selon l'une des revendications 1 à 12, caractérisé en ce que ladite polymérisation est réalisée en plusieurs étapes comprenant la préparation d'une semence suivie d'un ajout progressif du reste des produits.

14. Microdispersions de particules acryliques susceptibles d'être obtenues par le procédé selon l'une des revendications 1 à 13.

15. Utilisation des microdispersions obtenues ou susceptibles d'être obtenues selon le procédé de l'une des revendications 1 à 13, pour préparer des microgels.

16. Microgels, caractérisés en ce qu'ils sont obtenus à partir des microdispersions selon la revendication 14 par transfert des particules constituant lesdites microdispersions dans un solvant du PMMA, soit après séchage préalable desdites microdispersions, soit par ajout de ce même solvant à ladite microdispersion préalablement concentrée.

17. Microgels selon la revendication 16, caractérisés en ce que ledit solvant du PMMA est choisi dans le groupe des solvants aromatiques, des solvants chlorés tels que le chloroforme ou le chlorure de méthylène, des cétones et des esters, tels que acétate d'alkyle en C₂ à C₄.

18. Microgels selon l'une des revendications 16 ou 17, caractérisés en ce que ledit solvant du PMMA est l'acétate de butyle ou l'acétate d'éthyle.

19. Microgels selon l'une des revendications 16 à 18, caractérisés en ce que la concentration pondérale en polymère dans le solvant de PMMA est inférieur à 30 %, de préférence comprise entre 10 et 20 %.

20. Compositions cosmétiques, notamment vernis à ongles, caractérisées en ce qu'elles contiennent des microdispersions obtenues ou susceptibles d'être obtenues selon le procédé de l'une des revendications 1 à 13 ou des microgels selon l'une des revendications 16 à 19.

21. Composition selon la revendication 20, caractérisée en ce qu'elle contient moins de 30 % en poids, de préférence de 5 à 20 % en poids de microgel.

## Patentansprüche

1. Verfahren zur Herstellung einer stabilen Mikrodispersion von aus Acryl-Polymeren bestehenden Teilchen in einem organischen Lösungsmittel, das als Reaktionsmedium verwendet wird, in Anwesenheit eines aus einem Blockcopolymer bestehenden Stabilisators, dadurch gekennzeichnet, daß das Acryl-Polymer durch radikalische Polymerisation zumindest eines Acryl-Monomers in Anwesenheit eines Blockcopolymers auf der Basis von Methylpolymethacrylat (MPMA) und tert.Butylpolyacrylat (tBuPA) erhalten wird.

2. Verfahren nach Anspruch 1, dadurch gekennzeichnet, daß die Molmasse des Blockcopolymers zwischen 20 000 und 500 000, vorzugsweise zwischen 40 000 und 200 000 liegt.

3. Verfahren nach einem der Ansprüche 1 oder 2, dadurch gekennzeichnet, daß das Blockcopolymer ein Zweiblockcopolymer ist, und daß der tBuPA-Block 10 bis 90 Masse-%, vorzugsweise etwa 50 Masse-% des Copolymers beträgt.

4. Verfahren nach einem der Ansprüche 1 oder 2, dadurch gekennzeichnet, daß das Blockcopolymer ein Copolymer vom Dreiblock-Typ tBuPA-b-MPMA-b-tBuPA ist.

5. Verfahren nach einem der Ansprüche 1 bis 4, dadurch gekennzeichnet, daß das Acryl-Monomer ein Alkylacrylat oder ein Alkylmethacrylat, wobei die Alkyle lineare oder verzweigte C₁-C₁₈-Alkyle sind, oder eine Mischung dieser Monomere ist.

6. Verfahren nach einem der Ansprüche 1 bis 5, dadurch gekennzeichnet, daß das Acryl-Polymer ein Copolymer zumindest eines Acryl-Monomers und eines bifunktionellen Monomers ist.

7. Verfahren nach Anspruch 6, dadurch gekennzeichnet, daß das bifunktionelle Monomer Butandioldimethacrylat (BDMA) ist.

8. Verfahren nach einem der Ansprüche 1 bis 7, dadurch gekennzeichnet, daß die Menge an Blockcopolymer zwischen 1 und 50 Masse-%, vorzugsweise zwischen 5 und 30 %, bezogen auf die Gesamtheit der zur Herstellung des Acryl-Polymers verwendeten Monomere, beträgt.

9. Verfahren nach einem der Ansprüche 1 bis 8, dadurch gekennzeichnet, daß das Lösungsmittel ein (insbesondere C₁-C₄)-Alkohol, eine Alkohol-Mischung, insbesondere eine Mischung von Ethanol und Isopropanol, oder ein an Alkohol reiches Medium, beispielsweise eine Mischung von Ethanol und Butylacetat, oder ein beliebiges Lösungsmittel ist, das die tBuPA-Kette des Stabilisator-Copolymers solubilisiert und die MPMA-Kette desselben Copolymers sowie das Acryl-Polymer ausfällt.

10. Verfahren nach Anspruch 9, dadurch gekennzeichnet, daß das Lösungsmittel Ethanol ist.

11. Verfahren nach einem der Ansprüche 1 bis 10, dadurch gekennzeichnet, daß die radikalische Polymerisation in Anwesenheit eines Initiators, der zur Azo- und Peroxid-Familie gehört, durchgeführt wird.

12. Verfahren nach Anspruch 11, dadurch gekennzeichnet, daß der Initiator Azobisisobutyronitril oder Benzoylperoxid ist.

13. Verfahren nach einem der Ansprüche 1 bis 12, dadurch gekennzeichnet, daß die Polymerisation in mehreren Stufen durchgeführt wird, welche die Herstellung eines Primärprodukts, gefolgt vom progressiven Zusatz von Produkten, umfassen.

14. Mikrodispersionen von Acryl-Teilchen, die durch das Verfahren nach einem der Ansprüche 1 bis 13 erhalten werden können.

15. Verwendung von Mikrodispersionen, die gemäß dem Verfahren nach einem der Ansprüche 1 bis 13 erhalten werden oder erhalten werden können, bei der Herstellung von Mikrogelen.

16. Mikrogele, dadurch gekennzeichnet, daß sie aus Mikrodispersionen nach Anspruch 14 durch den Transfer von diese Mikrodispersionen bildenden Teilchen in einem MPMA-Lösungsmittel, entweder nach vorheriger Trocknung der Mikrodispersionen oder durch Zusatz desselben Lösungsmittels zur vorher konzentrierten Mikrodispersion, erhalten werden.

17. Mikrogele nach Anspruch 16, dadurch gekennzeichnet, daß das MPMA-Lösungsmittel ausgewählt ist aus der Gruppe aromatischer Lösungsmittel, chlorierter Lösungsmittel, wie Chloroform oder Methylenchlorid, Ketonen und Estern, wie C₂-C₄-Alkylacetat.

18. Mikrogele nach einem der Ansprüche 16 oder 17, dadurch gekennzeichnet, daß das MPMA-Lösungsmittel Butylacetat oder Ethylacetat ist.

19. Mikrogele nach einem der Ansprüche 16 bis 18, dadurch gekennzeichnet, daß die Massekonzentration von Polymer im MPMA-Lösungsmittel weniger als 30 %, vorzugsweise zwischen 10 und 20 % beträgt.

20. Kosmetische Zusammensetzungen, insbesondere Nagellack, dadurch gekennzeichnet, daß sie Mikrodispersionen, die gemäß dem Verfahren nach einem der Ansprüche 1 bis 13 erhalten werden oder erhalten werden können, oder Mikrogele nach einem der Ansprüche 16 bis 19 enthalten.

21. Zusammensetzung nach Anspruch 20, dadurch gekennzeichnet, daß sie zumindest 30 Masse-%, vorzugsweise 5 bis 20 Masse-%, Mikrogel enthält.

## Claims

1. A method of preparing a stable microdispersion of particles consisting of acrylic polymers in an organic solvent used as the reaction medium, in the presence of a stabilizer consisting of a block copolymer, characterized in that said acrylic polymer is obtained by the free-radical polymerization of at least one acrylic monomer in the presence of a block copolymer based on polymethyl methacrylate (PMMA) and polytert-butyl acrylate (PtBuA).

2. A method according to claim 1 characterized in that the molecular weight of said block copolymer is between 20,000 and 500,000, preferably between 40,000 and 200,000.

3. A method according to one of claims 1 or 2 characterized in that the block copolymer is a two-block copolymer and in that the PtBuA block represents 10 to 90% by weight, preferably of the order of 50%, of said copolymer.

4. A method according to one of claims 1 or 2 characterized in that said block copolymer is a copolymer of the PtBuA-b-PMMA-b-PtBuA three-block type.

5. A method according to one of claims 1 to 4 characterized in that said acrylic monomer is an alkyl acrylate or an alkyl methacrylate, said alkyls being linear or branched C₁ to C₁₈ alkyls, or a mixture of said monomers.

6. A method according to one of claims 1 to 5 characterized in that the acrylic polymer is a copolymer of at least one acrylic monomer and a difunctional monomer.

7. A method according to claim 6 characterized in that the difunctional monomer is butanediol dimethacrylate (BDMA).

8. A method according to one of claims 1 to 7 characterized in that the amount of block copolymer is between 1 and 50% by weight, preferably between 5 and 30%, based on all the monomers used to prepare said acrylic polymer.

9. A method according to one of claims 1 to 8 characterized in that the solvent is an alcohol, particularly a C₁ to C₄ alcohol, a mixture of alcohols, particularly a mixture of ethanol and isopropanol, or an alcohol-rich medium, for example a mixture of ethanol and butyl acetate or any solvent which solubilizes the PtBuA chain of the stabilizing copolymer and precipitates the PMMA chain of this same copolymer and the acrylic polymer.

10. A method according to claim 9 characterized in that said solvent is ethanol.

11. A method according to one of claims 1 to 10 characterized in that said free-radical polymerization is carried out in the presence of an initiator belonging to the family of azo compounds and peroxides.

12. A method according to claim 11 characterized in that said initiator is azobisisobutyronitrile or benzoyl peroxide.

13. A method according to one of claims 1 to 12 characterized in that said polymerization is carried out in several steps comprising the preparation of a seed followed by the gradual addition of the remaining products.

14. Microdispersions of acrylic particles obtainable by the method according to one of claims 1 to 13.

15. Use of the microdispersions obtained or obtainable by the method of one of claims 1 to 13 for the preparation of microgels.

16. Microgels, characterized in that they are obtained from the microdispersions according to claim 14 by transferring the constituent particles of said microdispersions into a solvent for PMMA, either after prior drying of said microdispersions or by adding this same solvent to said microdispersion concentrated beforehand.

17. Microgels according to claim 16 characterized in that said solvent for PMMA is selected from the group comprising aromatic solvents, chlorinated solvents such as chloroform or methylene chloride, ketones and esters such as a C₂ to C₄ alkyl acetate.

18. Microgels according to one of claims 16 or 17 characterized in that said solvent for PMMA is butyl acetate or ethyl acetate.

19. Microgeis according to one of claims 16 to 18 characterized in that the concentration by weight of polymer in the solvent for PMMA is less than 30%, preferably between 10 and 20%.

20. Cosmetic compositions, especially nail varnishes, characterized in that they contain microdispersions obtained or obtainable by the method of one of claims 1 to 13, or microgels according to one of claims 16 to 19.

21. A composition according to claim 20 characterized in that it contains less than 30% by weight, preferably from 5 to 20% by weight, of microgel.
